# EUROPEAN PATENT SPECIFICATION

(11) **EP 3 485 748 B1**
(45) Date of publication and mention of the grant of the patent: **21.10.2020**
(21) Application number: 18208681.9
(22) Date of filing: 03.06.2014
(51) Int. Cl.: A24F 47/00

(54) **ELECTRONIC CIGARETTE WITH IMPROVED SAFETY**
ELEKTRONISCHE ZIGARETTE MIT VERBESSERTER SICHERHEIT
CIGARETTE ÉLECTRONIQUE À SÉCURITÉ AMÉLIORÉE

(30) Priority: 03.06.2013 IT MI20130903; 03.06.2013 IT MI20130211 U; 03.06.2013 IT MI20130212 U; 04.07.2013 IT MI20131118
(43) Date of publication of application: 22.05.2019
(62) Divisional of application: 14744154.7
(73) Proprietor: Old Navigators Limited, Hong Kong (CN)
(72) Inventor: LUCIANI, Massimiliano, I-20093 COLOGNO MONZESE (Milan) (IT)
(74) Representative: Lunati & Mazzoni S.r.L.

(56) References cited:
- EP-A1- 1 618 803
- WO-A1-2013/020220

## Description

The present invention relates to an electronic cigarette with improved safety of the type specified in the preamble of the first claim.

Electronic cigarettes are currently known, which are increasingly common due to growing prohibitions and the negative effects on health of smoking cigarettes of the traditional type or the like.

Electronic cigarettes are in fact devoid of the combustion step of nicotine. The latter causes, in fact, various adverse effects on the nicotine molecule itself which have repercussions on the health of the smoker. The combustion step also causes odours which may prove unpleasant and which result, along with the health-related problems, in bans on smoking traditional cigarettes worldwide.

Electronic cigarettes, instead, are supplied with a liquid substance, in particular liquid nicotine or other substances. They further comprise a fluid atomizer, which acts by means of electric heaters or similar devices. The atomized fluid, i.e. fragmented into minute particles, for example, evaporated or the like, is then inhaled by the user during use of said electronic cigarette.

Given the alleged absence of the harmful effects mentioned and the possibility of use indoors, electronic cigarettes have in recent years, proved highly successful.

The prior art described above has several significant drawbacks.

In particular, electronic cigarettes contain liquid nicotine, in a solution or mixed with other substances, in an easily accessible chamber. As known, nicotine in the liquid state is very dangerous and must be handled with extreme caution, for example wearing special gloves.

In addition, it is very difficult to fill the chamber of the electronic cigarette with nicotine or similar substances. For said filling it is in fact necessary to unscrew the components of the cigarette and expose the chamber at least partially.

It is no coincidence that the use of electronic cigarettes is prohibited to those aged under 18. In fact, even the mere presence of electronic cigarettes near children can lead to serious accidents.

EP 1 618 803 A1 discloses an electronic cigarette according to the preamble of independent claim 1.

In this situation the technical purpose of the present invention is to devise an electronic cigarette with improved safety able to substantially overcome the drawbacks mentioned above.

Within the sphere of said technical purpose one important aim of the invention is to make an electronic cigarette which is safe and prevents contact of the user with the nicotine.

The technical purpose and specified aims are achieved by an electronic cigarette with improved safety according to the appended independent claim.

Preferred embodiments are described in the dependent claims.

The characteristics and advantages of the invention are clearly evident from the following detailed description of a preferred embodiment thereof, with reference to the accompanying drawings, in which:
**Fig. 1** shows an electronic cigarette with one portion enlarged;
**Fig. 2a** shows a detail of the electronic cigarette in a particular condition of use; and
**Fig. 2b** shows an accessory connected to the electronic cigarette,
**Fig. 3a** shows a detail of an embodiment of the electronic cigarette in a particular condition of use;
**Fig. 3b** shows the detail of Fig 3a in another particular condition of use;
**Fig. 4** shows an electronic cigarette according to the invention;
**Fig. 5a** shows another embodiment of the electronic cigarette in a first configuration;
**Fig. 5b** shows an embodiment in another configuration;
**Fig. 6** shows a further embodiment of the electronic cigarette;
**Fig. 7** shows another example of an electronic cigarette;
**Fig. 7a** shows a detail of the example of Fig 7;
**Fig. 8** shows a last example of an electronic cigarette;
**Fig. 8a** shows a detail of the example of Fig 8.

With reference to said drawings, reference numeral **1** globally denotes the electronic cigarette.

It comprises, in brief, a mouthpiece **4** through which the user inhales the atomised substance, a chamber **2** for storing a liquid substance to be inhaled, an atomizer **3** suitable to vaporize said substance to be inhaled, to produce a vaporized substance and to deliver said vaporized substance to said mouthpiece 4, an electric battery **5** connected to the atomizer 3 and suitable to permit the activation thereof and activating means **7** of the atomizer 3.

In detail, the electronic cigarette 1 preferably has a tubular shape and has the mouthpiece 4 at one end and the electric battery 5 at the other. The latter occupies a significant longitudinal section of the cigarette 1.

The atomizer 3 is in fluidic through connection with the chamber 2 to deliver the liquid substance to be inhaled to be atomized, and with the mouthpiece 4, for the ejection of the atomized substance to the user who inhales it.

The fluidic through connection between atomizer 3 and chamber 2 is preferably implemented by means of a wick **3a** or similar means, suitable to allow the passage of liquid and not of gaseous substances.

Diversely, the fluidic through connection between atomizer 3 and mouthpiece 4 is realised by means of ducts **3b** or the like.

The atomizer 3 further comprises at least one atomizing chamber **3c,** comprising an electrical resistor and possible filters or membranes, suitable to atomize, i.e. to divide into fine particles, the substance to be inhaled which was in the liquid state thereby creating a gaseous substance.

Structurally, the atomizer 3 is preferably placed inside the chamber 2, and the latter constitutes part of the external structure of the electronic cigarette 1. In particular, the chamber 2 comprises at least one outer wall **2a** bordering with the external environment.

The chamber 2 further comprises a vent suitable to allow the entry of air from the outside into the chamber 2 only in order to avoid problems of depression and the like.

The activation means 7 preferably comprise an activation button **7a,** suitable to control the activation of the atomizer 3, or even, or alternatively, sensors suitable to perceive the inhalation of the user and to command the activation of the atomizer 3 accordingly. The activation means 7 lastly preferably comprise a microprocessor or the like suitable to manage the functioning of the sensors and various means.

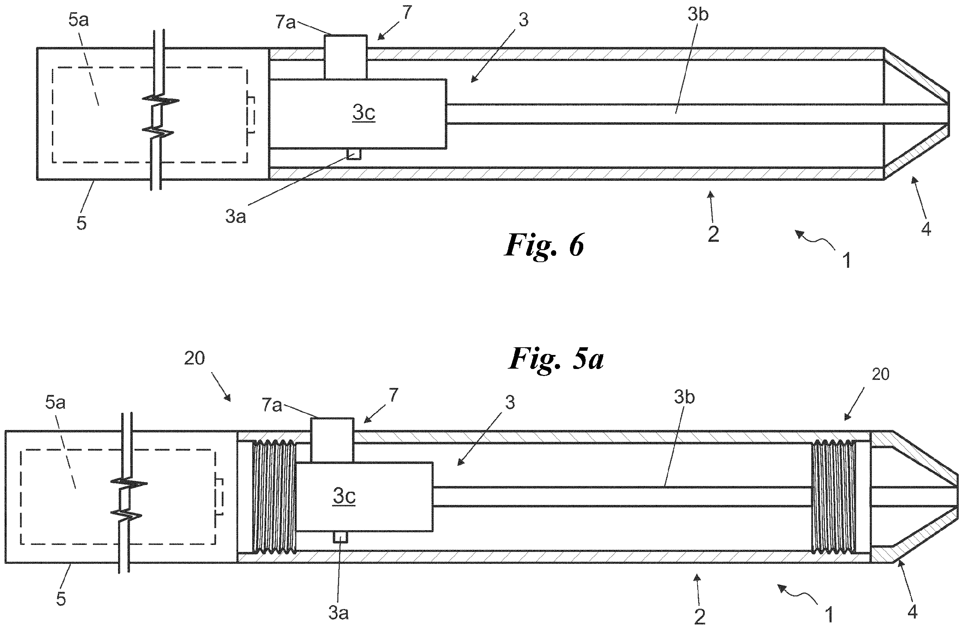

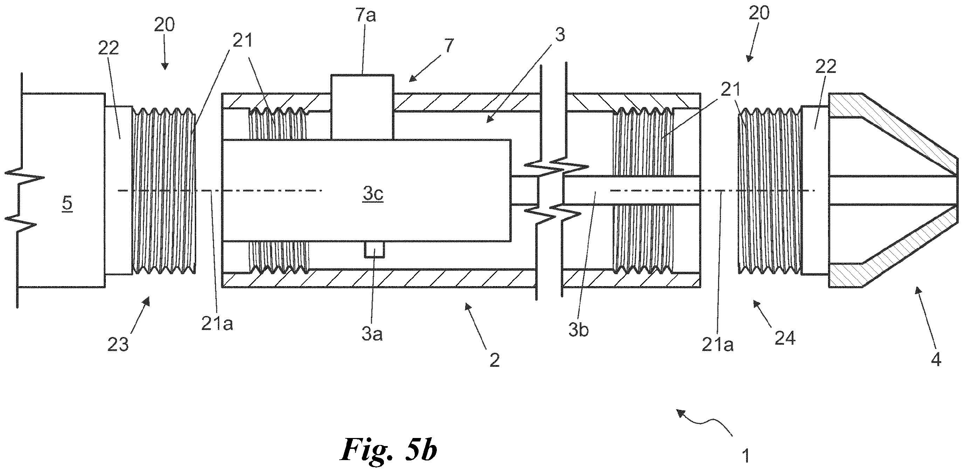

In particular, in the embodiments illustrated in Figs. 5a and 5b the electronic cigarette 1 comprises connection means **20** of the chamber 2 to the remaining part of said electronic cigarette 1. The connection means 20 are preferably suitable to make the liquid substance to be inhaled inside the chamber 2 accessible, for example for filling or replacement of said chamber 2.

In particular, the said connection means 20 comprise a first connection **23** which connects the chamber 2 and the casing 5 of the electric battery 5a suitable to make the liquid substance to be inhaled inside the chamber 2 accessible.

The connection means 20 may further comprise a second connection **24** between the chamber 2 and the mouthpiece 4 suitable to make the liquid substance to be inhaled inside the chamber 2 accessible.

Appropriately only one of the two connections is present and makes the substance to be inhaled inside the chamber 2 accessible. For example, only the first connection 23 may be present, while for example a threaded connection is present between the chamber 2 and the mouthpiece 4 to allow the replacement of the latter. For example, only the first connection 23 may be present, while for example a threaded connection is present between the chamber 2 and the mouthpiece 4, which does not allow access to the substance to be inhaled but which has the sole purpose of permitting the replacement of the latter. Vice versa only the second connection 24 may be present, while a threaded connection is present between the chamber 2 and the casing 5, which does not allow access to the substance to be inhaled but which has the sole purpose of permitting the replacement of the electric battery 5a.

Alternatively both connections 23 and 24 are present and make the substance to be inhaled inside the chamber 2 (Fig. 5a) accessible.

Obviously, in cases in which the connections do not allow access to the substance to be inhaled inside the chamber 2 they are not considered part of the connection means 20 for the purpose of this patent text and in particular the embodiments in Figs. 5a, 5b and 6.

Preferably, the cigarette 1 does not comprise further connection means 20 with the chamber 2, except, of course, in the case of forcing or breaking the chamber 2 or the other components.

Moreover, advantageously, the connection means 20 are safety connection means. Preferably all the connections composing the connection means 20 are safety connections.

In particular, the connection means 20 comprise at least one releasable "child-proof" connection.

The term "child-proof" refers to the so-called safety caps or similar in themselves know by the term "child-proof" for the experts in the field of containers for hazardous liquids, such as medicines, detergents and the like. The term "child-proof" is thus perfectly clear and unambiguous to the person skilled in the art.

Such caps are for example described in the patent documents classified in the international class of patents B65D 50/00 or more specific.

In detail, such caps, which also fall into the category of "child-proof", are caps requiring a double opening action, for example of unscrewing and pressing.

The safety connection means are also, generally, double action connection means thus for example comprising a hook and a thread or otherwise. The term double action is also understood to mean multiple action connection means, with more than two hooks or the like.

The connection means 20 comprise a releasable connection comprising a thread **21,** defining an axis **21a** preferably coincident with the main extension axis of the electronic cigarette 1, and a safety element **22** suitable to allow the release of the connection means 20 only if the unscrewing action is accompanied by a second action.

In a first example, the safety element 22 is of the elastic type and suitable to exert a distancing or in any case a failed interaction between the thread and counter thread make up the thread 21. It is therefore possible to release such connection means 20 exerting a said second thrust action in an axial direction in opposition to the safety element 22.

In a second example, the safety element 22 is of the elastic type and diametrically distances a gripping element needed to unscrew the thread 21. By exerting a second thrust action in the diametrical direction towards the axis 21a it is possible to lock the gripping element and the thread 21 and open the connection means 20.

In the alternative in Fig 6, the connection means 20 comprise only non-releasable connections, except of course in the case of tampering with the cigarette 1 or incorrect and non-intuitive use thereof, given by its shape and features. These therefore consist of gluing or welding.

In this case the liquid to be inhaled inside the chamber 2 is not accessible and the cigarette 2 is therefore substantially of the disposable type.

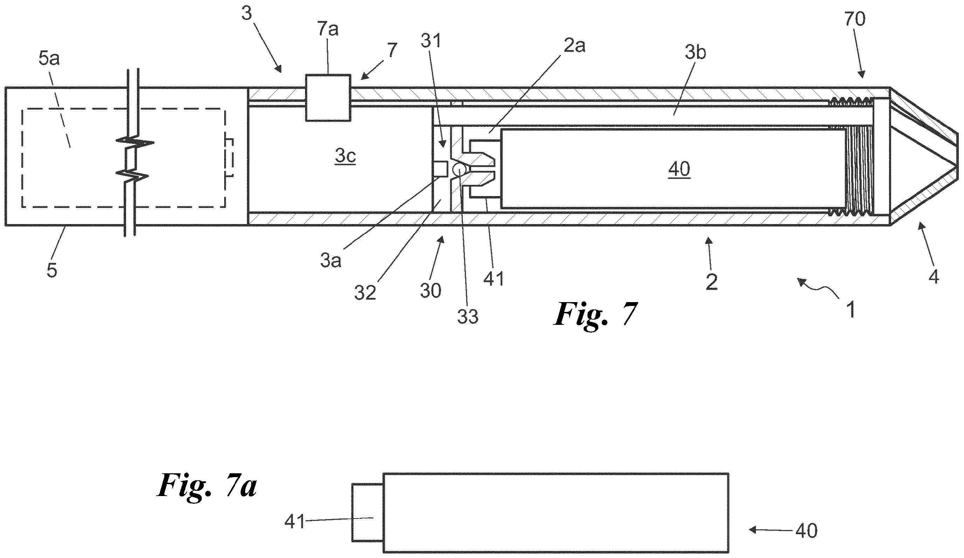

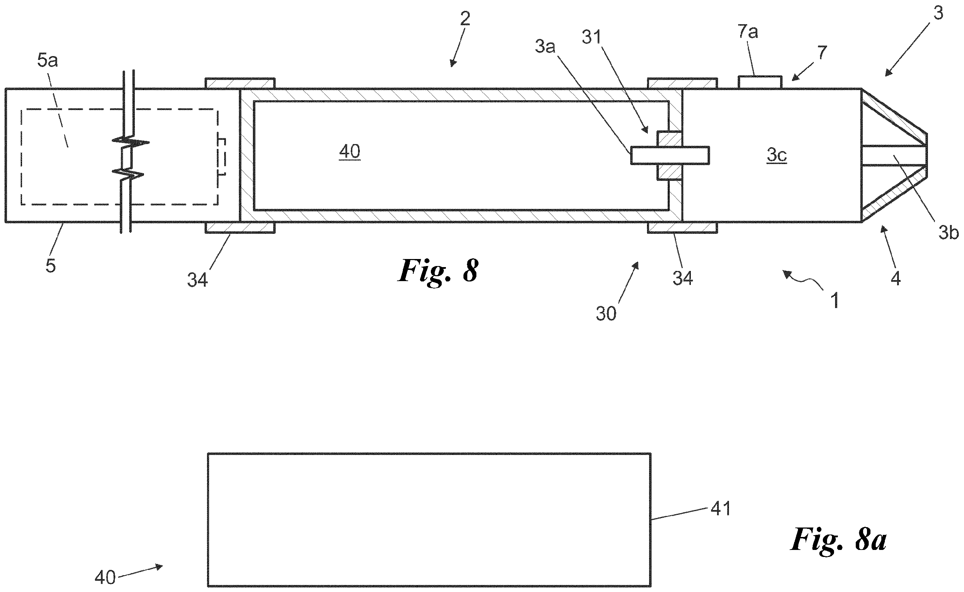

Alternatively, as illustrated in Figs. 7, 7a, 8, 8a, the chamber 2 is suitable to house, and thus preferably houses or comprises, a disposable cartridge **40** including the liquid substance to be inhaled.

The disposable cartridge 40 is for example of the type used for ink refills in fountain pens and includes a normally closed valve **41.** The normally closed valve 41 is suitable to prevent the leakage of the liquid substance to be inhaled when the cartridge 40 is not inserted in the cigarette. This valve 41 may also consist of a weakening of part of the cartridge 40, suitable to be broken when inserted in the cigarette 1, a valve similar to the valve present in the refill cartridges fountain pens or the like.

The electronic cigarette 1 thus comprises connection means **30** between said disposable cartridge 40 and the atomizer 3 suitable to connect the disposable cartridge 40 to the electronic cigarette 1 and to place the liquid substance to be inhaled contained in the disposable cartridge 40 in fluidic through connection with the atomizer 3.

In particular, the fluidic passage can be guaranteed by the wick 3a only or the like, or even by a chamber **32** suitable to contain a portion of the liquid substance to be inhaled and preferably fitted with a one-way valve **33** suitable to allow the entry of fluid into the chamber 32 but not the exit.

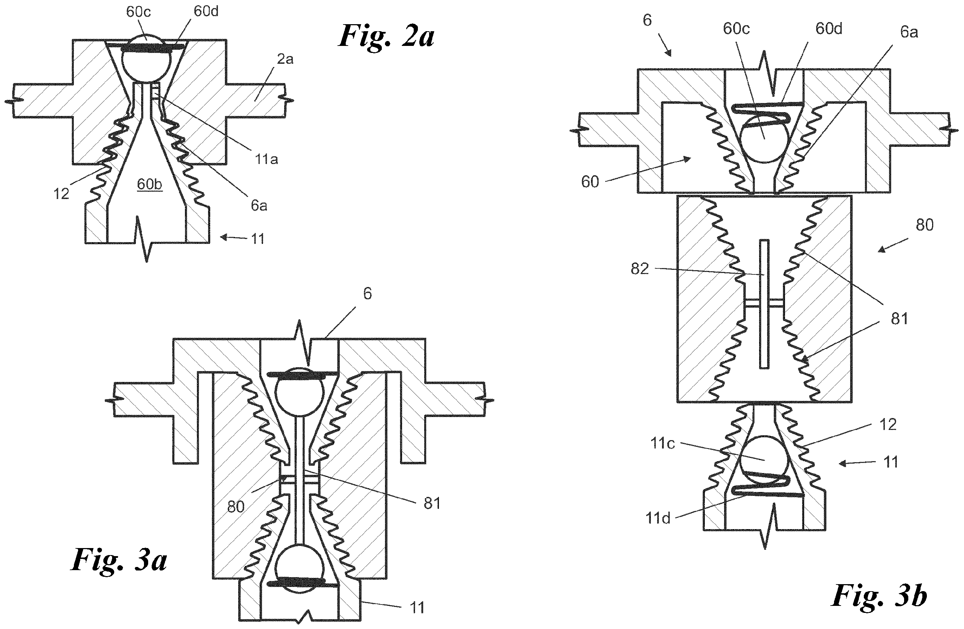

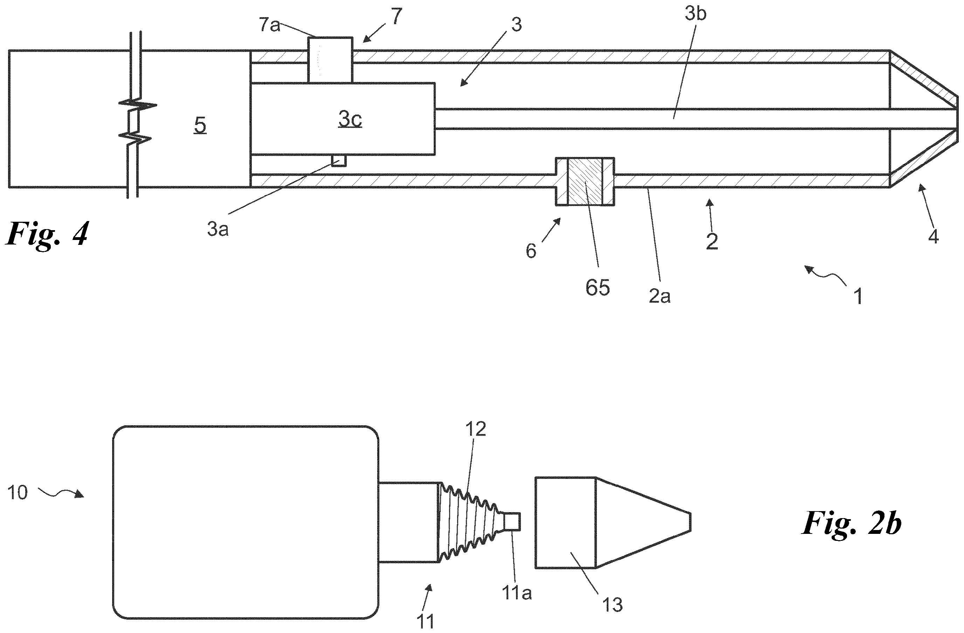

The connection means 30 further comprise an opening device **31** of the normally closed valve 41 suitable to open the normally closed valve 41 when the disposable cartridge 40 is connected to the connection means 30. The device 31 is thus preferably a portion of tube provided with a sharp surface, suitable to open the valve 41. The connection means 30 may also comprise further connection elements **34** of the mechanical, adhesive or similar type, for example threads (Fig. 2). These further connection elements 34 may also be arranged entirely or partly on the cartridge 40. They are suitable to mechanically connect the cartridge 40, which constitutes the chamber 2, to the cigarette 1.

In order to house the cartridge 40, the chamber 2 may comprise an internal volume **2a** (Fig. 7) and, in such case, the disposable cartridge 40 is housed in the internal volume 2a. In this case moreover the cigarette 1 comprises constraint means **70** of the chamber 2 to the remaining part of the electronic cigarette 1. The constraint means 70 are preferably suitable to make the internal volume 2a accessible for insertion or replacement of the cartridge 40. Advantageously, the constraint means 70 are safety connection means. Preferably all the connections composing the constraint means 70 are safety connections. In particular, the constraint means 70 comprise at least one releasable "child-proof" connection. The term "child-proof" refers to the so-called safety caps or similar in themselves know by the term "child-proof' for the experts in the field of containers for hazardous liquids, such as medicines, detergents and the like. The term "child-proof" is thus perfectly clear and unambiguous to the person skilled in the art. Such caps are for example described in the patent documents classified in the international class of patents B605D 50/00 or more specific. In detail, such caps, which also fall into the category of "child-proof', are caps requiring a double opening action, for example of unscrewing and pressing. Alternatively, the disposable cartridge 40 constitutes part of the outer surface of the electronic cigarette 1 (Fig. 8). In the latter case, the chamber 2 is consequently substantially entirely composed of the disposable cartridge 40. Moreover, again in the present case, the connection elements 34 are preferably safety connection means of the type previously described in relation to the constraint means 70.

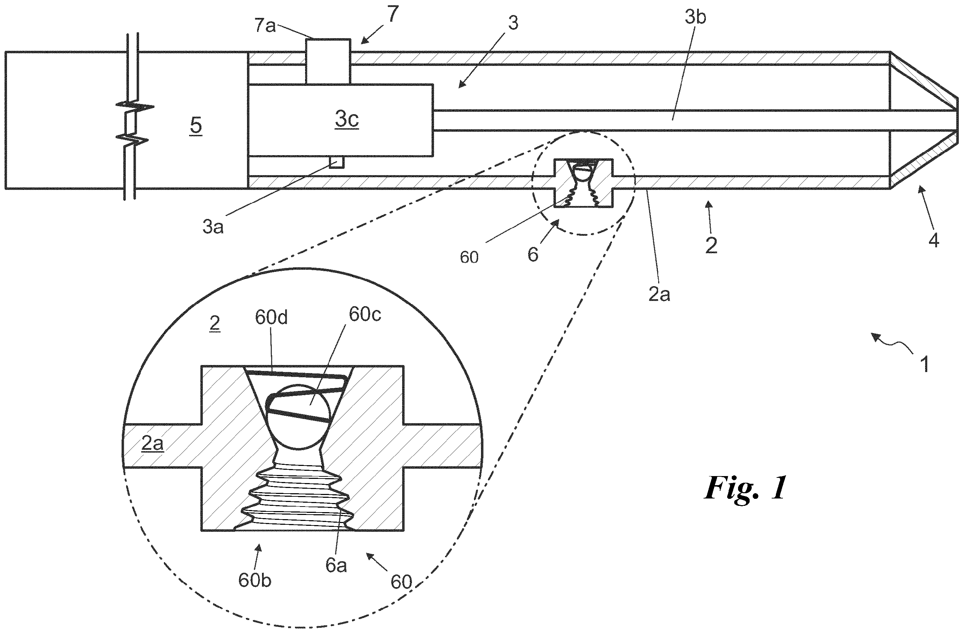

The electronic cigarette 1 also comprises means of access **6** to the chamber 2, arranged along the outer wall 2a. Such access means 6 are better illustrated in Figs. 1, 2a, 2b, 2c, 3 and 4.

Said access means 6 are of the one-way type, suitable to connect the external environment to the chamber 2 to permit the introduction of the liquid substance to be inhaled from the external environment.

They are composed (Figs. 1, 2a, 3a, 3b) of a one-way filling valve **60** and are not forming part of the invention.

The one-way means of access 6 are (Fig. 4) composed of a pierceable membrane **65,** suitable to permit the introduction of the liquid substance to be inhaled from the external environment by piercing means such as a syringe with needle including the liquid substance to be inhaled or the like.

The pierceable membrane 65 is in itself known and used for medical containers, ink containers, test tubes and containers of chemicals or the like.

It is made of a solid but partially fluid material suitable to return to its original shape after piercing, and thus to close again, such as a colloidal solution, a gel or the like. Or it is composed of a series of discs which may align or not to define a passage for a needle or the like, or so forth. Preferably the pierceable membrane has the property of being easily pierceable.

The pierceable membrane 65 is preferably a cylinder shape with a radial axis in relation to the cigarette 1 and surrounded by a portion of wall having the shape of a cylindrical ring and in highly resistant material, in particular in transparent polymer material.

The access means 6 are moreover accessible preferably by means of a special bottle **10** suitable to contain the store of the liquid substance to be inhaled.

In particular, the electronic cigarette 1 is part of a kit for inhalation **20** comprising the electronic cigarette 1 itself and the bottle 10.

The access means 6 are thus appropriately accessible substantially exclusively through the bottle 10. The term substantially exclusively, used in the claims and in the following text, means, of course, that it is impossible to prevent the access occurring by forcing the valve or other portions of the electronic cigarette 1 in a manner not specified in the instructions. It is therefore understood that if the electronic cigarette 1 is used correctly according to the manual, for example which comes with it when sold, the valve 6 is accessible exclusively through the bottle 10. In detail, the bottle 10 includes a neck **11** fitted with an opening **11a** for the exit of the liquid substance to be inhaled and a cap **13** to obstruct the opening 11a and close the bottle 10. The cap 10 is preferably of the type so-called "safety" or "child-proof" type, in themselves known.

In order to allow access, said means 6 comprise first releasable retaining means **6a** suitable to firmly connect the neck 11 of the bottle 10 to the access means 6 to connect in fluidic through connection the opening 11a of the bottle 10 with the access means 6.

Consequently, the bottle 10 comprises second releasable retaining means **12** suitable to connect the bottle 10 to the electronic cigarette 1 and in particular to the first releasable retaining means 6a. Said first and second releasable retaining means 6a and 12 are preferably, in the example of Figs. 1-2b threads and counter-threads arranged on a conical surface of the neck 11 and on a conical cavity **60b** positioned on the outer wall 2a.

The liquid substance to be inhaled may then be introduced into the chamber 2 preferably, and more preferably substantially exclusively, after the neck 11 has been placed in fluidic through connection with the chamber 2 through the valve 60 and after having connected the neck 11 to the valve 60 through the first and second releasable retaining means 6a and 12.

More in detail the valve 60 comprises an entry-only opening which preferably opens in the direction from the external environment to the chamber 2 in response to a mechanical thrust, for example by means of a tip, in the same direction. For example, the valve 60 may be a classic ball valve comprising a ball **60c** and elastic means **60d** which push it from the inside of the chamber 2 outwards (enlargement in Fig 1).

Consequently, the insertion of the neck 11 in the cavity 60b, and the constraint by using the means 6a and 12, achieves the pressure of the neck 11 on the valve 6 and the opening of the latter, as illustrated in Figure 2a.

In another example, Figs. 3a and 3b, both the bottle 10 and the valve 60 are suitable to allow the transit of fluid only when mutually connected.

To such purpose, the system may comprise a connector **80,** suitable to connect the neck 11 and the valve 60 and allow the transit of fluid by the same.

In this case, both the neck 11 and the valve 60 may comprise a entry-only opening which preferably opens in the direction from the external environment to the chamber in response to a mechanical thrust, for example by means of a tip, in the same direction. For example, the valve 60 may be of the type described above and illustrated in Figure 2a, while the neck 11 may include a ball **11c** and elastic means **11d** which push it from the inside of the bottle 10 outwards (enlargement in Figs. 3a and 3b).

The connector 80 may thus comprise third releasable retaining means **81** such as threads coupling to the second retaining means 12 of the bottle 10 and with the first releasable retaining means 6a of the means of access 6. It further comprises a thrust element **82** suitable to open the valves only when the connector 80 is connected (Fig. 3a).

The connector 80 may also be incorporated and/or in one piece with the neck 11 or the valve 60.

In the embodiment according to the invention in Fig 4, the cigarette 1 is preferably refilled with a special bottle fitted with a needle output for the containment and ejection of the liquid substance to be inhaled. In particular, the needle is preferably of the "hook", type i.e. is not straight but curves with an angle, between entry and exit, preferably greater than 90 ° and more preferably between 160 ° and 200 °, more preferably of amplitude approximately of a flat angle. This expedient prevents accidental releases of liquid from the bottle.

The bottle also has a closure of the child-proof type. A needle adaptor may instead be provided for, and preferably hook-shaped as previously mentioned, for bottles having a different attachment, such as those in the previous example (Figs. 1-3). In addition, preferably, the electronic cigarette 1 does not contain further openings of the chamber 2 to the outside or contains openings of the "safety" or "child-proof" type, i.e in particular openings that require a dual action of pressure and unscrewing to achieve opening.

The functioning of the electronic cigarette 1, the bottle 10 and the kit 20, described above in structural sense, is as follows.

The electronic cigarette 1 is first filled by means of the bottle 10 through the access means 6.

In detail the neck of the bottle 11 is connected, via the second releasable retaining means 12 to the first releasable retaining means 6a. The neck 11, inserted in the cavity 60a, thus pushes the ball 60c in the example of Fig 2 into the open position, in contrast to the elastic means 60d. The fluidic through connection between the inside of the bottle 10 and the inside of the chamber 2 is then opened in a safe manner and the liquid substance to be inhaled can be poured into the chamber 2. After filling is complete it is sufficient to unscrew the neck 11 from the cavity 6, mutually releasing the restraining means 6a and 12, to close the valve 6 automatically.

The bottle 10 is then also closed by means of the cap 13. The bottle 10 preferably further comprises a locking membrane, which makes it possible to pour the contents only by pressing on the container.

In the embodiment according to the invention in Fig 4, the electronic cigarette 1 is first filled by means of a syringe or a special container including a needle or a similar means of perforation of the access means 6 consisting of the pierceable membrane 65. The membrane is then perforated and the liquid substance to be inhaled is introduced into the chamber 2.

After filling is complete, it is sufficient to simply remove the needle or the like from the pierceable membrane 6 for it to close itself automatically.

After the safe and automatic closing of the chamber 2 it is possible to use the electronic cigarette 1, in the same way as the known electronic cigarettes, and thus in the same modalities.

The invention achieves some important advantages. In fact, the electronic cigarette 1 is completely safe both during filling and during use or mere storage.

For example, for a child it is impossible to access the contents of the chamber.

Variations may be made to the invention described herein without departing from the scope of the invention described in the appended claims. All details may be replaced with equivalent elements and the materials, shapes and dimensions may be any within the scope of the appended claims.

## Claims

1. Electronic cigarette (1) with improved safety comprising: a mouthpiece (4) through which the user inhales, a chamber (2) for storing a liquid substance to be inhaled, an atomizer (3) suitable to vaporize said substance to be inhaled and to produce a vaporized substance, said atomizer (3) being suitable to deliver said vaporized substance to said mouthpiece (4), an electric battery (5) connected to said atomizer (3) and suitable to permit the activation thereof, activating means (7) of said atomizer (3), said chamber (2) comprising: at least one outer wall (2a) forming a boundary with the external environment, and means of access (6) arranged along said outer wall (2a), said means of access (6) being of the one-way type suitable to connect the external environment with said chamber (2) and to permit the introduction of said liquid substance to be inhaled from the external environment, **characterised in that** said means of access (6) consist of a pierceable membrane (65) placed along said outer wall (2a) and said pierceable membrane consists of a membrane suitable to permit the introduction of the liquid substance to be inhaled from the external environment by piercing means and suitable to resume its original shape after being pierced.

2. Electronic cigarette (1) according to claim 1, wherein said pierceable membrane (65) consists of a solid but partially fluid membrane.

3. Electronic cigarette (1) according to claim 2, wherein said pierceable membrane (65) consists of a colloidal solution.

4. Electronic cigarette (1) according to claim 2, wherein said pierceable membrane (65) consists of a gel.

5. Electronic cigarette (1) according to claim 1, wherein said pierceable membrane (65) consists of a series of discs which may align or not to define a passage for a needle.

6. Kit for inhaling vaporized substances, comprising an electronic cigarette (1) according to one of the previous claims and a bottle (10) comprising a neck (11) provided with an aperture (11a) through which said liquid substance to be inhaled is able to flow.

## Patentansprüche

1. Elektronische Zigarette (1) mit verbesserter Sicherheit, umfassend: ein Mundstück (4) zum Ansaugen von Seiten eines Benutzers, einen Behälter (2) zum Speichern einer zu inhalierenden Substanz in flüssigem Zustand, einen Verdampfer (3), der geeignet ist, die genannte zu inhalierende Substanz zu verdampfen und eine verdampfte Substanz zu erzeugen, wobei der genannte Verdampfer (3) geeignet ist, die genannte verdampfte Substanz zu dem genannten Mundstück (4) zu leiten, eine an den genannten Verdampfer (3) angeschlossene elektrische Batterie (5), die geeignet ist, die Aktivierung desselben zu gestatten, Aktivierungsmittel (7) des genannten Verdampfers (3), wobei der genannte Behälter (2) Folgendes umfasst: mindestens eine an die Außenumgebung angrenzende Außenwand (2a) und entlang der genannten Außenwand (2a) angeordnete Zugangsvorrichtungen (6), wobei die genannten Zugangsvorrichtungen (6) des Typs mit nur einem Weg und geeignet sind, die Außenumgebung mit dem genannten Behälter (2) zu verbinden und die Einleitung der genannten zu inhalierenden Substanz in flüssigem Zustand aus der Außenumgebung zu gestatten, **dadurch gekennzeichnet, dass** die genannten Zugangsvorrichtungen (6) aus einer entlang der genannten Außenwand (2a) angeordneten perforierbaren Membran (65) bestehen und die genannte perforierbare Membran aus einer Membran besteht, die geeignet ist, das Einleiten der zu inhalierenden Substanz in flüssigem Zustand aus der Außenumgebung über Perforierungsmittel zu gestatten und geeignet ist, nach der Perforierung wieder in ihre ursprüngliche Form zurückzukehren.

2. Elektronische Zigarette (1) nach Anspruch 1, bei der die genannte perforierbare Membran (65) aus einem festen doch teilweise fließenden Material besteht.

3. Elektronische Zigarette (1) nach Anspruch 2, bei der die genannte perforierbare Membran (65) aus einer kolloidalen Lösung besteht.

4. Elektronische Zigarette (1) nach Anspruch 2, bei der die genannte perforierbare Membran (65) aus einem Gel besteht.

5. Elektronische Zigarette (1) nach Anspruch 1, bei der die genannte perforierbare Membran (65) aus einer Reihe von Scheiben besteht, die eventuell miteinander ausgerichtet werden können und so einen Durchgang für eine Nadel definieren.

6. Kit zum Inhalieren von verdampften Substanzen, umfassend eine elektronische Zigarette (1) nach einem der vorangegangenen Ansprüche und einen Flakon (10), umfassend ein mit einer Öffnung (11a) zum Austreten der genannten zu inhalierenden Substanz in flüssigem Zustand ausgestattetes Mundstück (11).

## Revendications

1. Cigarette électronique (1) à sécurité améliorée comprenant : un embout (4) pour l'aspiration par un utilisateur, un réservoir (2) pour le stockage d'une substance à inhaler à l'état liquide, un atomiseur (3) apte à atomiser ladite substance à inhaler et à produire une substance atomisée, ledit atomiseur (3) étant apte à acheminer ladite substance audit embout (4), une batterie électrique (5) connectée audit atomiseur (3) et apte à permettre l'activation de ce dernier, des moyens d'activation (7) dudit atomiseur (3), ledit réservoir comprend : au moins une paroi extérieure (2a) limitrophe de l'environnement extérieur et des moyens d'accès (6) disposés le long de la paroi extérieure (2a), lesdits moyens d'accès (6) étant du type à une seule voie aptes à connecter l'environnement extérieur audit réservoir (2) et à permettre l'introduction de ladite substance à inhaler à l'état liquide de l'environnement extérieur, **caractérisée en ce que** lesdits moyens d'accès (6) sont constitués d'une membrane perforable (65) disposée le long de ladite paroi extérieure (2a), et ladite membrane perforable est constituée d'une membrane apte à permettre l'introduction de la substance à inhaler à l'état liquide de l'environnement extérieur à travers des moyens de perforation et apte à reprendre la forme d'origine après la perforation.

2. Cigarette électronique (1) selon la revendication 1, dans laquelle ladite membrane perforable (65) est constituée d'une matière solide mais partiellement fluide.

3. Cigarette électronique (1) selon la revendication 2, dans laquelle ladite membrane perforable (65) est constituée d'une solution colloïdale.

4. Cigarette électronique (1) selon la revendication 2, dans laquelle ladite membrane perforable (65) est constituée d'un gel.

5. Cigarette électronique (1) selon la revendication 1, dans laquelle ladite membrane perforable (65) est constituée d'un ensemble de disques pouvant s'aligner ou pas en définissant un passage pour une aiguille.

6. Kit per inhalation de substances atomisées, comprenant une cigarette électronique (1) selon l'une des revendications précédentes et un flacon (10) comprenant un bec (11) doté d'une ouverture (11a) pour la sortie de ladite substance à inhaler à l'état liquide.
